# EUROPEAN PATENT APPLICATION

(11) **EP 1 094 077 A1**
(43) Date of publication of application: **25.04.2001**
(21) Application number: 99928208.0
(22) Date of filing: 30.06.1999
(51) Int. Cl.: C07K 14/745, C07K 1/22, C07K 16/18, C12P 21/02

(54) **NOVEL SUGAR CHAIN-BONDED THROMBOMODULIN-LIKE PEPTIDE**

(30) Priority: 30.06.1998 JP 19965498
(71) Applicant: MOCHIDA PHARMACEUTICAL CO., LTD., Shinjuku-ku Tokyo 160-8515 (JP)
(72) Inventor: HASE, Sumihiro, Ashiya-shi, Hyogo 659-0032 (JP); WAKABAYASHI, Hiroyuki, Mochida Pharma. Co., Ltd., Tokyo 160-8515 (JP)
(74) Representative: Grünecker, Kinkeldey, Stockmair & Schwanhäusser Anwaltssozietät
(86) International application number: JP9903532
(87) International publication number: WO0000516

(57) **Abstract**

[Subject] A peptide which has pharmaceutically preferable properties and has a thrombomodulin activity, and a purification method and a detection method thereof are obtained.

[Means for Solution] A novel peptide which has a novel sugar chain and has a thrombomodulin activity, and a pharmaceutical composition which contains it as the active ingredient are provided. A method for separating said peptide by affinity chromatography with anti-HNK-1 antibody and an enzyme immunoassay method with anti-HNK-1 antibody are also provided.

## Description

### Technical Field

This invention relates to a novel peptide which has a novel sugar chain and has a thrombomodulin activity. More particularly, the invention relates to a peptide which contains a 3-position-O-sulfated glucuronic acid β1-3 galactose β1-3 galactose β1-4 xylose chain (to be referred to as 3SAGGX chain hereinafter) and has a thrombomodulin activity.

The invention also relates to a peptide which binds specifically to anti-human natural killer cell-1 antibody (to be referred to as anti-HNK-1 antibody hereinafter) and has a thrombomodulin activity.

The invention also relates to a pharmaceutical composition which contains this peptide as the active ingredient or a method for treating thrombosis and the like diseases, which comprises administering this pharmaceutical composition.

The invention also relates to a purification method and a detection method of this peptide.

The invention also relates to the 3SAGGX chain released and purified from this peptide and to a reducing end-modified product thereof.

### Background Art

Thrombomodulin has been reported by Esmon *et al*. in 1981 (*Proceedings of the National Academy of Sciences of the USA*, 78, 2249 - 2254, 1981) as a protein having a unique property to convert thrombin from coagulation-tropic enzyme to anti-coagulation enzyme, which is present on the surface of endothelium. They have succeeded in isolating and purifying it from rabbit lung tissue and reported the results in the succeeding report (*The Journal of Biological Chemistry*, 257, 859 - 864, 1982). Thereafter, total cDNA sequence of human thrombomodulin and its amino acid sequence have been reported (*EMBO Journal*, 6, 1891 - 1897, 1987; *Biochemistry*, 26, 4350 - 4357, 1987) and the role of each domain is being revealed. It is considered that thrombomodulin removes the blood coagulation action of thrombin by binding to thrombin, and the thrombin-thrombomodulin complex shows anti-coagulation action by the activating of protein C. That is, since thrombomodulin has a possibility of exerting both of blood coagulation inhibition action and of fibrinolysis enhancing action, its clinical applications are expected.

In general, antithrombin III and heparin having anti-blood coagulation action or urokinase, streptokinase, tissue plasminogen activator and the like substances having thrombolytic action are used as therapeutic agents for diseases caused by abnormal blood coagulation ability. However, these substances have side effects such as bleeding tendency, and their actions have a partiality to either anti-blood coagulation or thrombolysis. In consequence, great concern has been directed toward clinical applications of thrombomodulin which has a possibility of exerting both of these actions or thrombomodulin-like substances that basically have thrombomodulin activities in which they have the affinity for thrombin and enhances activation of protein C.

With regard to the preparation of such thrombomodulin or thrombomodulin-like substances, the following examples have so far been reported. In this connection, unless otherwise noted, the molecular weight is shown by a value measured under non-reducing condition by sodium dodecyl sulfate-polyacrylamide gel electrophoresis (to be referred to as SDS-PAGE hereinafter).

P.W. Majerus *et al*. have purified thrombomodulin from human placenta and reported that its molecular weight was 75 K (*The Journal of Biological Chemistry*, 259, 12246 - 12251, 1984). Maruyama *et al*. have purified thrombomodulin from human lung and reported that its properties were identical to those of placenta thrombomodulin (*Journal of* *Clinical Investigation*, 75, 987 - 991, 1985) In addition, Suzuki *et al*. have partially purified thrombomodulin from human platelets, determined its molecular weight to be 78 K and reported that the thrombomodulin preparations from platelets, placenta and lung endothelium have mutually identical properties based on their electrophoresis behavior, affinity for thrombin and substrate affinity for protein C (*The Journal of Biochemistry*, 104, 628 - 632, 1988).

Regarding studies carried out by employing gene engineering techniques, Suzuki et al. have cloned a signal peptide-containing human thrombomodulin precursor gene from a human lung cDNA library, elucidated the total gene structure and thereby revealed its amino acid sequences of 575 amino acid residue continued from an 18 amino acids signal peptide (*EMBO Journal*, 6, 1891 - 1897, 1987). Thereafter, Suzuki *et al*. have reported that the human thrombomodulin prepared by means of gene engineering has the same activity of human thrombomodulin purified from a natural tissue (*The Journal of Biological Chemistry*, 264, 4872 - 4876, 1989) and that the human thrombomodulin activity is limited to a sequence of from the 345 to 462 position amino acid residues counting from the amino-terminal, and the activity is lost even a part of the moiety is deleted (*The Journal of Biological Chemistry*, 264, 10351 - 10353, 1989; and Abstract of Papers, the 12th Meeting of International Society on Thrombosis and Hemostasis, p. 334, Subject No. 1039, 1989).

In addition, regarding thrombomodulin-like substances having thrombomodulin activity, there are known natural types such as human urine soluble thrombomodulin substances (JP-A-63-30423 (the term "JP-A" as used herein means an "unexamined published Japanese patent application"), JP-A-63-146898, JP-A-3-86900, JP-A-3-218399, and Yamamoto *et al*., *The Journal of Biochemistry*, 113, 433 - 440, 1993).

Examples of such substances obtained by means of gene engineering techniques include soluble thrombomodulin preparations from which the transmembrane region and intracytoplasmic region are removed (JP-A-1-6219, JP-A-2-255699, JP-A-3-133380, JP-A-3-259084, JP-A-4-210700, JP-W-3-503757 (the term "JP-W" as used herein means an "unexamined published Japanese international patent application"), JP-W-4-505554, EP 474273, WO 91/04276, WO 91/05803, WO 91/15514, WO 92/00325, WO 92/03149, WO 93/15755, and Doi *et al*., Abstract of Papers No. 3, the 113th Annual Meeting of Pharmaceutical Society of Japan, Subject No. 30EM14-1, 1993).

It has been reported that the amount of soluble thrombomodulin in blood or urine changes in certain diseases and morbid states, so that its usefulness as a marker of such diseases and morbid states is suggested. For example, it has been reported that the amount of thrombomodulin in blood increases in disseminated intravascular coagulation syndrome (to be referred to as DIC hereinafter), systemic lupus erythematosus, thrombotic thrombocytopenia purpura, articular rheumatism, progressive scleroderma, diabetes mellitus, renal disorders, adult respiratory distress syndrome, lung diseases such as pulmonary thromboembolism (*Rinsho Kensa* (Clinical Inspection), vol. 33, pp. 1643 - 1647, 1989), gestational toxicosis (*Ketsueki to Myakukan* (Blood and Vessel), vol. 19, p. 490, 1988), liver diseases (*Ketsueki to Myakukan*, vol. 20, p. 407, 1989), Kawasaki Disease (*Journal of Japanese Society on Thrombosis and Hemostasis*, vol. 3, p. 343, 1992), chronic glomerulonephritis (*Journal of Japanese Society on Thrombosis and Hemostasis*, vol. 3, p. 349, 1992), after periodic hemodialysis (*Journal of Japanese Society on Thrombosis and Hemostasis*, vol. 1, p. 395, 1990), aging (*Journal of The Japan Hematological Society*, vol. 54, p. 286, 1991) and the like cases. Also, it has been reported that thrombomodulin in urine decreases by renal insufficiency (*Rinsho Ketsueki* (Clinical Blood), vol. 31, p. 1191, 1990).

It is considered that N-type sugar chains and O-type sugar chains can be linked to these thrombomodulin or thrombomodulin-like substances, based on their amino acid sequences. However, reports on the actually linked sugar chains are extremely scarce, merely including information on N-type sugar chains reported by Niimi *et al*. (*Iyakuhin Kenkyu* (Medicament Research), vol. 28, no. 12, pp. 863 - 873, 1997).

As extremely small number of reports on the O-type sugar chains of thrombomodulin, WO 91/04276 discloses a recombinant soluble thrombomodulin which has sugar chains containing chondroitin and/or chondroitin sulfate, WO 91/05803 discloses a recombinant soluble thrombomodulin which is modified with sulfated glycosaminoglycan and JP-A-4-210700 discloses a recombinant soluble thrombomodulin to which sulfated glycosaminoglycan is not linked.

On the other hand, Yamamoto *et al*. (*The Journal of Biochemistry*, 113, 433 - 440, 1993) and Nakano *et al*. (*Thrombosis and Haemostasis*, 79, 331 - 337, 1998) described that chondroitin sulfate does not exist in soluble thrombomodulin prepared from human urine. However, it is the present situation that details of the O-type sugar chains, particularly on its structures and the like, are not sufficiently analyzed.

On the other hand, glucuronic acid β1-3 galactose β1-3 galactose β1-4 xylose chain without 3-O-sulfation is known as a linkage region when a glycosaminoglycan chain is linked to a peptide, but there is no report on a sugar chain consisting of just only four sugars in which the 3-position of glucuronic acid is O-sulfated.

### Disclosure of the Invention

In order to apply the blood coagulation controlling action or platelet agglutination controlling action of thrombomodulin or thrombomodulin-like substances to medicaments, great concern has been directed toward the provision of a peptide which can be purified to a higher purity and detected easily in biological samples such as blood, urine and the like of administered patients.

An object of the invention is to provide a peptide having thrombomodulin activity, which can be purified to a higher purity and detected easily in biological samples such as blood, urine and the like of administered patients, and a pharmaceutical composition which contains it as the active ingredient.

Another object of the invention is to provide a purification method, a detection method and a detection kit of the just described peptide of the invention.

In the course of intensive studies on thrombomodulin-like substances, the inventors of this invention have unexpectedly found that some of thrombomodulin-like substances can bind specifically to anti-HNK-1 antibody and, as a result of further studies, have succeeded unexpectedly in isolating a novel thrombomodulin-like peptide which has a novel sugar chain, namely a 3SAGGX chain, in which its terminal glucuronic acid is O-sulfated. Also, we have found that said peptide is useful as a medicament. In addition, we have found a method for purifying the thrombomodulin-like substance having this 3SAGGX chain to high purity and a method for detecting the same. This invention has been accomplished on the basis of these findings.

The first embodiment of the invention is a peptide which contains a 3SAGGX chain and has a thrombomodulin activity. Regarding said peptide, a peptide which is soluble in aqueous solution is preferable, a peptide which is soluble in aqueous solution in the absence of nonionic surface active agents is more preferable, and a peptide having an amino acid sequence of the 1 to 494 position amino acid residues of Sequence ID No. 1, having an amino acid sequence of the 1 to 498 position amino acid residues of Sequence ID No. 2, or having an amino acid sequence obtained by applying substitution, addition, insertion and/or deletion of one or several amino acids to said amino acid sequence is more preferable.

The second embodiment of the invention is a peptide which binds specifically to anti-HNK-1 antibody and has a thrombomodulin activity. Regarding said peptide, a peptide which does not substantially contain a peptide to which chondroitin and/or chondroitin sulfate is linked is preferable. Regarding said peptide, a peptide which is soluble in aqueous solution is preferable, a peptide which is soluble in aqueous solution in the absence of nonionic surface active agents is more preferable, and a peptide having an amino acid sequence of the 1 to 494 position amino acid residues of Sequence ID No. 1, having an amino acid sequence of the 1 to 498 position amino acid residues of Sequence ID No. 2, or having an amino acid sequence obtained by applying substitution, insertion, addition and/or deletion of one or several amino acids to said amino acid sequence is more preferable.

The third embodiment of the invention is a pharmaceutical composition which contains a peptide that has a 3SAGGX chain and a thrombomodulin activity or a peptide that binds specifically to anti-HNK-1 antibody and has a thrombomodulin activity, as the active ingredient. Preferred examples of said peptide are the same as described in the first or second embodiment of the invention.

The fourth embodiment of the invention is a method for purifying a peptide having a 3SAGGX chain and a thrombomodulin activity or a peptide that binds specifically to anti-HNK-1 antibody and has a thrombomodulin activity, which is characterized by a separation method with the affinity of an antibody capable of specifically recognizing and binding to the 3SAGGX chain. Preferred antibody is anti-HNK-1 antibody, and it is more preferable to use an antibody affinity column with resin to which said antibody is linked.

The fifth embodiments of the invention are an enzyme immunoassay (to be referred to as EIA hereinafter) method and a kit for detecting a peptide having a 3SAGGX chain and a thrombomodulin activity or a peptide that binds specifically to anti-HNK-1 antibody and has a thrombomodulin activity, which uses an antibody capable of specifically recognizing and binding to the 3SAGGX chain. Preferred antibody is anti-HNK-1 antibody.

A further embodiment of the invention relates to a sugar chain obtained by releasing and purifying a 3SAGGX chain from glycopeptides and its reducing end-modified form. Regarding said sugar chain, sugar alcohol or glycopeptide to which serine linked to its reducing end is preferable.

### Best Mode for Carrying Out the Invention

The following describes the invention in detail.

Terms as used herein are described below.

The 3SAGGX chain is a 3-position-O-sulfated glucuronic acid β1-3 galactose β1-3 (± sialic acid α2-6) galactose β1-4 xylose chain, and it includes both cases in which sialic acid is linked to the third galactose from the terminal through α2-6 bond and in which it is not linked thereto. Since so-called glycosaminoglycan chain is not linked to glucuronic acid, it has a structure of only 4 sugars. It is considered that the xylose moiety is forming *O*-glycoside bond with the hydroxyl group of the 472-position and/or 474-position serine of the amino acid sequence, represented by the Sequence ID No. 1, of the peptide having a thrombomodulin activity.

The thrombomodulin activity means an action to bind to thrombin and thereby to enhance the activation of protein C by thrombin. Its binding to thrombin can be confirmed for example by its adsorption to an affinity column in which thrombin is used as the ligand. Its enhancing of the activation of protein C by thrombin can be confirmed by a method for measuring protein C activation ability in the presence of thrombin with a synthetic substrate Boc-Leu-Ser-Thr-Arg-MCA, which is disclosed for example in JP-A-3-218399. In addition, peptides having thrombomodulin activity are generally referred to as thrombomodulin-like peptides in which thrombomodulin itself is also included.

The enzyme immunoassay method (sometimes to be referred to as EIA hereinafter) with an antibody capable of specifically recognizing and binding to the 3SAGGX chain means a detection of peptides having the 3SAGGX chain with an antibody prepared by chemically binding peroxidase, galactosidase or the like enzyme to the antibody capable of specifically recognizing and binding to the 3SAGGX chain. An antibody which specifically recognizes 3-position-O-sulfated glucuronic acid as an antigen and binds thereto (e.g., anti-HNK-1 antibody) can be exemplified as a preferable antibody, but any other antibody can be used with the proviso that it can specifically recognize the 3SAGGX chain and bind thereto. By adding a substrate which develops color by enzyme reaction, the presence and the amount of the antigen of interest are measured based on the coloring degree. Any substance can be used, with the proviso that it shows enzyme concentration- and reaction time-dependent coloring strength and does not exert influences upon the measuring system. As occasion demands, the measurement can be carried out with more higher accuracy by combining it with another EIA which uses an antibody that recognizes another portion, than the 3SAGGX chain, of the peptide of the invention as an antigen and binds thereto.

The anti-HNK-1 antibody is an antibody which specifically recognizes an antigen of human natural killer cells, CD57, and binds thereto, and it specifically recognizes and binds to glucuronic acid whose 3-position is O-sulfated. The anti-HNK-1 antibody can be purchased as a commercially available polyclonal or monoclonal antibody (e.g., Anti-NK Cell: clone subclass (mouse) IgM, produced and supplied by Cosmo Bio, Code No. IT-0289) or prepared in accordance with a usual method.

The peptide of the invention having the 3SAGGX chain and thrombomodulin activity may be either a natural origin peptide or a product obtained as a recombinant peptide, with the proviso that it has the 3SAGGX chain and thrombomodulin activity, but a natural origin peptide is more preferable. In the case of the natural origin peptide, it may be either a human origin peptide or an animal origin peptide, but a human origin peptide is more preferable. An example suitable for use in medicaments is a soluble thrombomodulin, for example, a peptide having an amino acid sequence of the 1 to 494 positions of Sequence ID No. 1, a peptide having an amino acid sequence of the 1 to 498 position of Sequence ID No. 2, or a peptide obtained by applying substitution, deletion, addition and/or insertion of one or several amino acids to said peptide is preferable. Since the N-terminal has heterogeneity, a peptide which further has Phe-Pro- is also preferable. In the case of the recombinant form, peptides having an amino acid sequences obtained by applying substitution, deletion, addition and/or insertion of one or several amino acids to the 1 to 498 position amino acid residues of Sequence ID No. 2, within such a range that the modifications do not exert influences upon the 3SAGGX chain and thrombomodulin activity, are also included in the invention.

Also, the invention is peptides having thrombomodulin activity, which can be purified or detected by the use of an antibody capable of specifically recognizing the 3SAGGX chain and binding thereto.

The peptide of the invention which specifically binds to anti-HNK-1 antibody and has thrombomodulin activity may be either a natural origin peptide or a product obtained as a recombinant peptide, with the proviso that it specifically binds to anti-HNK-1 antibody and has thrombomodulin activity, but a natural origin peptide is more preferable. In the case of the natural origin peptide, it may be either a human origin peptide or an animal origin peptide, but a human origin peptide is more preferable. An example suitable for use in medicaments is a soluble thrombomodulin, for example, a peptide having an amino acid sequence of the 1 to 494 positions of Sequence ID No. 1 or a peptide having an amino acid sequence of the 1 to 498 positions of Sequence ID No. 2 is preferable. Since the N-terminal has heterogeneity, a peptide which further has Phe-Pro- is also preferable. In the case of the recombinant form, peptides having amino acid sequences obtained by applying substitution, deletion, addition and/or insertion of one or several amino acids to the 1 to 498 position amino acid residues of Sequence ID No. 2, within such a range that the modifications do not exert influences upon the 3SAGGX chain and thrombomodulin activity, are also included in the invention. Also preferred is a peptide which does not substantially contain a peptide to which chondroitin and/or chondroitin sulfate is linked.

The peptide of the invention which has the 3SAGGX chain and thrombomodulin activity can be obtained by a separation method with the affinity of an antibody capable of specifically recognizing and binding to the 3SAGGX chain. Though the starting material is not particularly limited, it is desirable that a further separation step making use of said affinity is applied on thrombomodulin or a soluble thrombomodulin purified from a human or animal origin natural source, such as human lung, human placenta, human urine, human blood or the like, in accordance with a usual method with anion exchange chromatography, affinity chromatography in which thrombin is used as the ligand, gel filtration chromatography or the like means, or on a recombinant thrombomodulin or recombinant soluble thrombomodulin prepared by means of gene engineering techniques, but it may also be carried out as an initial step or an intermediate step of the usual purification method or it may be repeated several times as occasion demands. Also, if necessary, it may be carried out in combination with cation exchange chromatography, hydrophobic chromatography, hydroxyapatite chromatography or the like means. In the case of preparing by means of gene engineering techniques, host cells are transformed with a vector capable of expressing a peptide having thrombomodulin activity in the usual way. Simultaneous transfection (cotransfection) of a vector capable of expressing a peptide having thrombomodulin activity into host cells together with a vector capable of expressing a peptide having glucuronyl transferase activity and/or a vector capable of expressing a peptide having sulfotransferase activity, e.g., HNK-1 sulfotransferase, is also a desirable method. Also, transformation of host cells can be effected with a vector capable of expressing both of a peptide having thrombomodulin activity and a peptide having glucuronyl transferase activity and/or a peptide having sulfotransferase activity, e.g., HNK-1 sulfotransferase.

Though not particularly limited, the antibody which specifically recognizes the 3SAGGX chain and binds thereto is preferably anti-HNK-1 antibody which is more preferably monoclonal antibody. In addition, in the separation method which uses the affinity of an antibody capable of specifically recognizing the 3SAGGX chain and binding thereto, said antibody can be binded to a carrier, such as agarose, cellulose, dextran or the like, and separation can be carried out by a column, batch or the like means of which a column method is particularly desirable.

When it is carried out by a column method, said antibody is linked to a carrier by cyanogen bromide and packed in a column, and sample solution is passed through the column to effect adsorption of a peptide having the 3SAGGX chain and thrombomodulin activity. Thereafter, said peptide is eluted by passing eluent through the column under such conditions that the affinity of thus adsorbed peptide for the antibody is attenuated. As occasion demands, it may be combined with thrombin affinity chromatography or gel filtration chromatography.

The thus obtained peptide of the invention can be made into more suitable condition as a medicament by inactivating viruses of treatment at 60 ± 2°C for 10 hours or removing viruses with a hollow fiber membrane.

Actions and properties of the peptide of the invention are shown in the following.

### Test Example 1 Structural analysis of the peptide of Inventive Example 1

### (1) Terminal amino acid sequence

The peptide obtained in Inventive Example 1 was subjected to reductive carboxymethylation in accordance with the method of C.H.W. Hirs *et al*. (*Methods in Enzymology* (C.H.W. Hirs ed.), Vol. 11, 199 - 203, Academic Press, New York, 1967) and then desalted to be used as a sample for amino acid sequence analysis.

The N-terminal amino acid sequence analysis was carried out by a gas phase amino acid sequence automatic analyzer (470A Type, manufactured by Applied Biosystems), the carboxy terminal (to be referred to as C-terminal hereinafter) amino acid was determined by the hydrazinolysis method, and the C-terminal amino acid sequence analysis was carried out by treating the sample with carboxypeptidase Y and quantitatively analyzing the thus released amino acids by an amino acid analyzer (manufactured by JASCO) with high performance liquid chromatography.

Results of the analysis of N-terminal side and C-terminal side amino acid sequences of the inventive peptide of Inventive Example 1 are shown below.
N-terminal: Ala-Pro-Ala-Glu-Pro-Gln-Pro-Gly-Gly-Ser-Gln-Cys-Val-Glu-His-Asp-Cys-Phe-Ala-Leu-Tyr-Pro-Gly-Pro-Ala-Thr-Phe-Leu-
C-terminal: -Val-Gly-Leu

As is evident from these results, N-terminal amino acid sequence of the peptide obtained in Inventive Example 1 completely coincides with the reports of human thrombomodulin so far revealed, but the C-terminal side amino acid sequence corresponds to a portion of the 492 to 494 position amino acid residues. That is, it was considered that the peptide of Inventive Example 1 is a peptide which has an amino acid sequence corresponding to the 1 to 494 position amino acid residues of human thrombomodulin.

### (2) Sugar chain analysis

The peptide obtained in Inventive Example 1 was subjected to hydrazinolysis (60°C, 50 hours) in accordance with the method of N. Kuraya and S. Hase (*The Journal of Biochemistry*, 112, 122 - 126, 1992) to liberate sugar chains which were then subjected to N-acetylation and pyridylamination to be used as a sample for sugar chain analysis (pyridylaminated sugar chains; to be referred to as PA-sugar chains hereinafter). PA-sugar chain containing xylose on reducing end side (side of binding to amino acid) were purified by gel filtration chromatography and reversed phase chromatography, and these structures were analyzed by analytical method based on the two dimensional sugar chain mapping method of S. Hase combined with exoglycosidase digestion (sequential use of β-glucuronidase and β-galactosidase) (*Methods in Enzymology* (Ginsburg, V. ed.), Vol. 230, 225 - 237, Academic Press, New York, 1994), methylation analysis, mass spectrometry and NMR analysis.

Results of the analysis of the xylose-containing sugar chains linked to the inventive peptide of Inventive Example 1 are shown below. In this connection, each monosaccharide was abbreviated as follows.
SO₄-3GlcA: 3-position-O-sulfated glucuronic acid
Gal: galactose
Sia: sialic acid
Xyl: xylose
   SO₄-3GlcA β1-3Gal β1-3(± Sia α2-6)Gal β1-4 Xyl

Based on these results, it was found that the xylose-containing sugar chain structures of sugar chains linked to the peptide obtained in Inventive Example 1 are new findings from the viewpoints that they are linked to the peptide with only four sugars structure of glucuronic acid β1-3 galactose β1-3 galactose β1-4 xylose, and that these are novel sugar chains so far unreported in terms that O-sulfate group is linked to the 3-position of glucuronic acid.

In addition, when, in accordance with the method of J. Suzuki et al. (*Agricultural and Biological Chemistry*, 55, 283 - 284, 1991), the peptide obtained in Inventive Example 1 was heated at 100°C for 3 hours in 4 M trifluoroacetic acid and then the thus released monosaccharides were subjected to pyridylamination and determined by ion exchange HPLC, 1 mole of xylose was detected per 1 mole of the peptide. Since xylose-containing sugar chains were not found other than the 3SAGGX chain based on these results, it was confirmed that 1 mole of the 3SAGGX chain is linked to 1 mole of the peptide.

### (3) Chondroitinase treatment

Molecular weight measured by SDS-PAGE and thrombomodulin activity were not changed by chondroitinase treatment. In consequence, it was confirmed again that chondroitin and/or chondroitin sulfate is not linked to the peptide of Inventive Example 1.

### Test Example 2 Activities of the peptide obtained in Inventive Example 1

Affinity for thrombin (anti-thrombin action), protein C activation ability in the presence of thrombin and anti-blood coagulation activity were measured by the methods described in JP-A-3-218399.

As the results, the peptide of the invention showed an action to bind to thrombin and thereby strongly inhibited its coagulation activity. Also, the peptide of the invention showed a strong ability to activate protein C in the presence of thrombin. The peptide of the invention also showed a strong action to prolong blood coagulation time.

### Test Example 3 Acute toxicity in mice

The peptide obtained in Inventive Example 1 or Inventive Example 2 was administered to five ddY male mice by intravenous injection and the animals were observed for 7 days, but significant toxicity and mortality were not found with a pharmaceutically effective dose.

### Test Example 4 Solubility

The peptide obtained in Inventive Example 1 or Inventive Example 2 dissolved in distilled water at least to a concentration of 30 mg/ml at room temperature.

As is evident from the aforementioned descriptions and tests, the peptide of the invention has the thrombomodulin activity and shows low toxicity, so that it is expected that this peptide is effective for the prevention and treatment of diseases in which blood coagulation exacerbation is concerned, such as DIC, thromboses, peripheral vessel obstruction, myocardial infarction, cerebral infarction, transient cerebral ischemic attack, gestational toxicosis, hepatic insufficiency and renal insufficiency. The peptide of the invention has the identical activities to those of known natural or recombinant thrombomodulin-like peptides.

The peptide of the invention can be made into pharmaceutical preparations, preferably injections, which are suited for effectively administering to patients, with an appropriate carrier or medium generally used in medicaments, such as sterile water, physiological saline, plant oil, nontoxic organic solvent or the like, if necessary in combination with a filler, a coloring agent, an emulsifying agent, a suspending agent, a stabilizing agent, a preservative and the like additives. When the peptide of the invention is used as injections, the preparation is administered to each patient by dividing it into 1 to 6 doses per day, in one portion or continuously. The daily dose is from 0.01 to 500 mg, preferably from 0.05 to 10 mg, as the peptide of the invention, though it can be optionally changed depending on the age, body weight, symptoms and the like of each patient.

In addition, the peptide of the invention can be bound or adsorbed to the surface of artificial blood vessels, artificial organs, catheters and the like medical devices by crosslinking agents and the like. By such applications, blood coagulation on the surface of medical devices can be prevented. It can also be used as a coagulation inhibitor at the external blood circulation.

### Examples

The following illustratively describes the peptide of the invention, its purification method and its detection method with reference to examples, but the invention is not limited to the following examples.

### Inventive Example 1 Purification of soluble thrombomodulin which specifically binds to anti-HNK-1 antigen, from human urine

100 liters of fresh urine of healthy males collected with antiseptics such as phenol were adjusted to pH 8.5 with 10% NaOH, and the thus formed precipitate was removed. Next, the thus treated urine was adjusted to pH 5.5 with 4 M HCl and filtered through acrylonitrile fibers to remove urine urokinase by its adsorption and then the passed urine was desalted and concentrated using an ultrafiltration membrane having a nominal cutoff molecular weight of 40,000. This was adjusted to pH 7.3 and then treated at 60°C for 15 minutes.

The concentrated urine was passed through a 300 ml column of DEAE cellulose (manufactured by Whatman) which had been conditioned in advance with 0.05 M phosphate buffer, pH 6.5, containing 0.068 M NaCl, thereby the active fraction was adsorbed, the column was washed with 750 ml of the same buffer used in the conditioning and then the active fraction was eluted with an acetate buffer, pH 4.0, containing 0.05 M NaCl.

The eluate was concentrated using an ultrafiltration membrane having a nominal cutoff molecular weight of 30,000, adjusted to pH 7.5 with 2 M NaOH and then passed through a 2.5 ml column of DIP-thrombin-agarose which had been conditioned in advance with 0.02 M Tris-HCl buffer, pH 7.5, containing 0.1 M NaCl, 1 mM benzamidine hydrochloride and 0.5 mM CaCl₂, thereby the active fraction was adsorbed. Next, the column was washed with 25 ml of the same buffer used in the conditioning, elution was carried out with 0.02 M Tris-HCl buffer, pH 7.5, containing 1 M NaCl, 1 mM benzamidine hydrochloride and 0.5 mM EDTA, and the eluate was dialyzed against the same buffer used in the conditioning and then re-purified by a DIP-thrombin-agarose chromatography under the same conditions as described above. In the second DIP-thrombin-agarose chromatography, the same volume column was used and washed with 10 ml of the buffer used in the conditioning and then washed with 10 ml of 0.02 M Tris-HCl buffer, pH 7.5, containing 0.8 M NaCl, 1 mM benzamidine hydrochloride and 0.5 mM CaCl₂, and the active fraction was then eluted with 0.02 M Tris-HCl buffer, pH 7.5, containing 1 M NaCl, 1 mM benzamidine hydrochloride and 0.5 mM EDTA.

The eluate was concentrated using an ultrafiltration membrane having a nominal cutoff molecular weight of 30,000 and passed through a 2.5 ml column of anti-HNK-1 antibody-agarose which had been conditioned in advance with 0.01 M phosphate buffer, pH 7.0, containing 0.14 M NaCl, thereby the active fraction containing the peptide of the invention was adsorbed. A thrombomodulin fraction was also obtained from the passed fractions. Thereafter, the column was washed with 25 ml of the same buffer used in the conditioning, elution was carried out with 0.02 M Tris-HCl buffer, pH 7.5, containing 1 M NaCl, 1 mM benzamidine hydrochloride and 0.5 mM EDTA, and then the eluate was dialyzed against the same buffer used in the conditioning to obtain the active fraction containing the peptide of the invention.

The active fraction containing the peptide of the invention finally obtained by the above production method was isolated as a peptide which showed a single band in SDS-PAGE.

### Inventive Example 2 Production of recombinant soluble thrombomodulin which specifically binds to anti-HNK-1 antigen

Recombinant soluble thrombomodulin was produced in accordance with the method of WO 92/00325. That is, a vector capable of expressing a soluble thrombomodulin composed of 498 amino acid residues having an Ala-Pro-Ala-amino acid sequence as its amino terminus (to be referred to as N-terminus hereinafter) was prepared from DNA obtained from a human placenta cDNA library, and this vector was transfected to CHO cells and then a high expression strain was obtained by gene amplification. Culture broth of this high expression strain was applied to DIP-thrombin-agarose column chromatography and gel filtration. Using the anti-HNK-1 antibody-agarose column described in Inventive Example 1, an active fraction containing the peptide of the invention was collected in the same manner. A thrombomodulin active fraction was also obtained from the fractions passed through the anti-HNK-1 antibody-agarose column.

### Inventive Example 3 EIA using anti-HNK-1 antibody

Anti-HNK-1 antibody (Cosmo Bio Co., Ltd., Code No. IT-0289) was dissolved in carbonate buffer (pH 9.6) to a concentration of 2 µg/ml, and 200 µl of this solution was added to and coated on a commercially available EIA plate. After standing at 4°C for 3 hours or more, this plate was coated with 200 µl of 3% bovine serum albumin-phosphate buffered saline and allowed to stand overnight at 4°C to carry out blocking of the plate. After washing of the plate, 100 µl of sample and standard solution of a peptide having the 3SAGGX chain and thrombomodulin activity were added thereto and allowed to react at 37°C for 2 hours. After washing, 100 µl of biotinated anti-HNK-1 antibody was added thereto to carry out the reaction at 37°C for 2 hours. After washing, 100 µl of avidin-peroxidase was added. After washing, 100 µl of o-phenylenediamine, 0.024% hydrogen peroxide solution was added as the substrate, and the reaction was carried out at room temperature for 10 minutes and then stopped by adding 50 µl of 1 N sulfuric acid. Absorbance at 492 nm was measured using a microplate reader, and the peptide having the 3SAGGX chain and thrombomodulin activity in the sample was determined from a standard curve. Said peptide was detected in the active fraction of Inventive Example 1 containing the peptide of the invention and purified to a level of about 4 times per protein before and after anti-HNK-1 antibody affinity chromatography.

### Inventive Example 4 EIA with anti-HNK-1 antibody

The 3SAGGX chain-linked thrombomodulin purified in Inventive Example 1 was administered to male rats by intravenous injection with a dose of 0.6 mg/kg. Blood samples were collected from caudal veins 1 minute and 60 minutes after the administration, blood plasma was separated and then the peptide having 3SAGGX chain and thrombomodulin activity was measured by EIA in accordance with the method of Inventive Example 3. As the result, its concentration was 16 µg/ml 1 minute after the administration and 4 µg/ml 60 minutes after the administration.

### Preparation Example 1

- Peptide of Inventive Example 1: 10 mg
- Purified gelatin: 50 mg
- Sodium phosphate: 34.8 mg
- Sodium chloride: 81.8 mg
- Mannitol: 25 mg

The above components were dissolved in distilled water for injection, and the solution was adjusted to a total volume of 10 ml, filter-sterilized, put into aseptic vials in 1.0 ml portions and then freeze-dried to prepare a freeze-dried composition.

### Preparation Example 2

- Peptide of Inventive Example 2: 20 mg
- Albumin: 20 mg
- Sodium phosphate: 34.8 mg
- Sodium chloride: 81.8 mg
- Mannitol: 25 mg

By weighing each of the above components, a freeze-dried preparation was obtained in the same manner as described in Preparation Example 1.

### Industrial Applicability

According to the peptide which has the 3SAGGX chain and thrombomodulin activity or the peptide which binds specifically to anti-HNK-1 antibody and has thrombomodulin activity, anti-HNK-1 antibody and the like antibodies specifically bind to said sugar chain of these peptides. In consequence, they can be purified to a high purity making use of a separation method which uses the affinity of an antibody that specifically recognizes the 3SAGGX chain and binds thereto, such as anti-HNK-1 antibody, for example by the use of an anti-HNK-1 antibody affinity column, so that the obtained peptide free from contaminated components is suitable for a medicament. It also is suitable for a standard substance of an enzyme immunoassay kit for the detection of a peptide having thrombomodulin activity, which uses an antibody that specifically recognizes the 3SAGGX chain and binds thereto, such as anti-HNK-1 antibody.

Since the peptide of the invention has the thrombomodulin activity and shows low toxicity, it is expected that this peptide is effective for the prevention and treatment of diseases in which blood coagulation exacerbation is concerned, such as DIC, thromboses, peripheral vessel obstruction, myocardial infarction, cerebral infarction, transient cerebral ischemic attack, gestational toxicosis, hepatic insufficiency and renal insufficiency. In addition, the peptide of the invention can be bound or adsorbed to the surface of artificial blood vessels, artificial organs, catheters and the like medical devices by crosslinking agents and the like. By such an application, blood coagulation on the surface of medical devices can be prevented. It can also be used as a coagulation inhibitor at the external blood circulation.

Also, when it is necessary to monitor concentration of a peptide having thrombomodulin activity, for example, at the process of its purification or in biological samples such as blood and urine of patients after administration of the peptide having thrombomodulin activity, it is possible to detect the peptide easily and accurately by EIA which uses an antibody that specifically recognizes the 3SAGGX chain and binds thereto, such as anti-HNK-1 antibody, so that this peptide is useful as a medicament in terms of the ability to prevent or alleviate generation of side effects and to be used economically.

Also, the 3SAGGX chain can be released as a sugar alcohol by treating the peptide of the invention with sodium hydroxide or the like alkali in the presence of sodium borohydride or the like reducing agent. Alternatively, it can be released as a glycopeptide in which the 3SAGGX chain is linked to serine, by subjecting the peptide of the invention to reductive carboxymethylation and then digesting it with a protease such as Actinase E (produced and supplied by Kaken Pharmaceutical). The thus released sugar alcohol or glycopeptide can be purified by anion chromatography or using affinity column of an antibody that specifically recognizes the 3SAGGX chain and binds thereto, such as anti-HNK-1 antibody, and further purification with gel filtration or amide column may be combined. The thus purified sugar alcohol or glycopeptide can be used as a standard substance of an enzyme immunoassay kit for the detection of a peptide having thrombomodulin activity, which uses an antibody that specifically recognizes the 3SAGGX chain and binds thereto, such as anti-HNK-1 antibody.

In addition, a separation method which uses the affinity of anti-HNK-1 antibody or the like antibody that specifically recognizes the 3SAGGX chain and binds thereto, such as a purifying method with an anti-HNK-1 antibody affinity column of a peptide having the 3SAGGX chain and thrombomodulin activity or a peptide that binds specifically to anti-HNK-1 antibody and has a thrombomodulin activity, and a detecting method or detecting kit of a peptide which has the 3SAGGX chain and thrombomodulin activity or a peptide that binds specifically to anti-HNK-1 antibody and has thrombomodulin activity, by an EIA which uses anti-HNK-1 antibody or the like antibody that specifically recognizes the 3SAGGX chain and binds thereto are also useful. When the EIA of the invention which uses an antibody that specifically recognizes the 3SAGGX chain and binds thereto, such as anti-HNK-1 antibody, is used in combination with an EIA which uses an antibody that recognizes another part of thrombomodulin than the 3SAGGX chain, such as a sequence of from the 345 to 462 position amino acid residues counting from the amino-terminal, as an antigen and binds thereto, it becomes to be possible to easily and accurately determine the peptide of the invention, a peptide which does not have the 3SAGGX chain but has thrombomodulin activity and a peptide which has the 3SAGGX chain but does not have thrombomodulin activity in blood, urine and the like biological samples of healthy people and patients.

## Claims

1. A peptide which contains a 3-position-O-sulfated glucuronic acid β1-3 galactose β1-3 galactose β1-4 xylose chain and has a thrombomodulin activity.

2. The peptide according to claim 1, wherein its amino acid sequence has an amino acid sequence of the 1 to 498 position amino acid residues of Sequence ID No. 2 or an amino acid sequence obtained by applying substitution, addition, insertion and/or deletion of one or several amino acids to said amino acid sequence.

3. The peptide according to claim 1 or 2, wherein its amino acid sequence has an amino acid sequence of the 1 to 494 position amino acid residues of Sequence ID No. 1.

4. A peptide which binds specifically to anti-human natural killer cell-1 antibody and has a thrombomodulin activity.

5. The peptide according to claim 4, wherein it does not substantially contain a peptide to which chondroitin and/or chondroitin sulfate is linked.

6. The peptide according to any one of claims 1 or 5, wherein said peptide is a natural origin peptide.

7. The peptide according to any one of claims 1 or 5, wherein said peptide is a recombinant peptide.

8. A pharmaceutical composition which contains the peptide described in any one of claims 1 to 7 as the active ingredient.

9. A method for purifying a peptide having thrombomodulin activity, which uses affinity of an antibody that specifically recognizes a 3-position-O-sulfated glucuronic acid β1-3 galactose β1-3 galactose β1-4 xylose chain and binds thereto.

10. The purification method according to claim 9, wherein said antibody is anti-human natural killer cell-1 antibody.

11. An enzyme immunoassay method for detecting a peptide having thrombomodulin activity, which uses an antibody that specifically recognizes a 3-position-O-sulfated glucuronic acid β1-3 galactose β1-3 galactose β1-4 xylose chain and binds thereto.

12. The detection method according to claim 11, wherein said antibody is anti-human natural killer cell-1 antibody.

13. An enzyme immunoassay kit for detecting a peptide having thrombomodulin activity, which contains anti-human natural killer cell-1 antibody.
